(19) **Europäisches Patentamt**

**European Patent Office**

**Office européen des brevets**

(11) **EP 0 709 058 B1**

(12) **EUROPEAN PATENT SPECIFICATION**

(45) Date of publication and mention
of the grant of the patent:
**26.02.2003   Bulletin 2003/09**

(51) Int Cl.⁷: **A61B 5/22**

(21) Application number: **95830451.1**

(22) Date of filing: **26.10.1995**

(54) **A fitness machine with monitoring of physical performance**

Fitnessgerät mit Überwachung des physischen Leistungsniveaus

Appareil d'exercice avec évaluation de la performance physique

(84) Designated Contracting States:
**DE FR GB IT NL**

(30) Priority: **28.10.1994   IT   BO940472**

(43) Date of publication of application:
**01.05.1996   Bulletin 1996/18**

(73) Proprietor: **TECHNOGYM S.p.A.**
**47035 Gambettola (Forli) (IT)**

(72) Inventor: **Alessandri, Nerio**
**I-47020 Longiano (Forli') (IT)**

(74) Representative: **Lanzoni, Luciano**
**c/o BUGNION S.p.A.**
**Via Goito, 18**
**40126 Bologna (IT)**

(56) References cited:
**WO-A-81/01507**          **DE-A- 3 920 526**
**US-A- 4 367 752**          **US-A- 4 911 427**

EP 0 709 058 B1

**Description**

The present invention relates to a fitness machine with monitoring of the athlete's physical performance, that is to say, a fitness machine which, by means of readings relative to the exercises performed on the machine, provides the user with a value which is a function of the degree of training performed.

The following description relates to an exercise machine for sports, although the present invention can be applied to rehabilitation machines; reference is, therefore, made to an athlete, this term denoting any user of sport or rehabilitation machines.

An important factor in sports and rehabilitation activities is the assessment of the results achieved, that is to say, the level of fitness.

For an examination of the performance of a given exercise or series of exercises, or rather the athlete's physical effort during the corresponding exertion, one of the parameters considered is the heart rate, that is to say, the heart beats per minute of the athlete during exercise.

The simple knowledge of the heart rate, as affirmed by current studies in sports medicine, is insufficient in order to ascertain an athlete's level of fitness, since it is also necessary to know the physical effort which induced such a rate.

In fact, only by comparing effort and heart rate is it possible to obtain the parameters which allow the athlete's level of fitness to be determined.

The problem of assessing effort is similar to a knowledge of the amount of oxygen consumed by the athlete during exercise, which is equally difficult to assess.

Many of the fitness machines currently manufactured are used to perform exercises designed to specifically improve the cardiac and respiratory systems. For these machines, a knowledge of the aerobic capacity, or the maximum quantity of oxygen that the organism can metabolise during a given period of time, is particularly important.

It is known that a well-planned training programme, performed using the afore-mentioned machines or with aerobic sports of the kind described below, allows an improvement in the athlete's aerobic capacity.

In other words, with the same heart rate, an athlete with greater aerobic capacity can consume a larger volume of oxygen in the given period of time, with the same number of heart beats, yet a higher work load.

Obviously, it is not easy to assess an athlete's level of fitness by considering his/her heart rate and a value relative to the physical effort exerted during exercise; moreover, the level of fitness cannot be "quantified" with an easily understandable value.

It is also known from document US 4 911 427, a fitness machine with monitoring of the athlete's physical performance constituted by a cyclette® and a transducer means designed to provide as output a signal relative to the speed applied by the user of the wheel of the cyclette.

A sensor which measure the heart rate of the user is connected to a processing means which provides a performance value which is a function of the signal relative to the speed performance, the heart rate, the predetermined parameters of the machine and the personal data of the user.

The Applicant has designed a fitness machine which allows an immediate assessment of the athlete's level of fitness, and which provides an easily understandable value.

Based on the fact that oxygen consumption is a function of the effort exerted, the Applicant has designed a machine which uses an algorithm in which the heart rate is considered relative to certain of the athlete's personal data, such as his/her weight and age, the combination of values used being suitably corrected so as to obtain a final value that is easily assessed.

The advantages of the invention are more clearly shown in the detailed description below, with reference to the accompanying drawings, which illustrate an embodiment by way of example only and in which:

- figure 1 is a block diagram of a possible embodiment of the present invention;
- figure 2 is a schematic illustration of an embodiment of the present invention, in which the fitness machine includes an exercise bike.

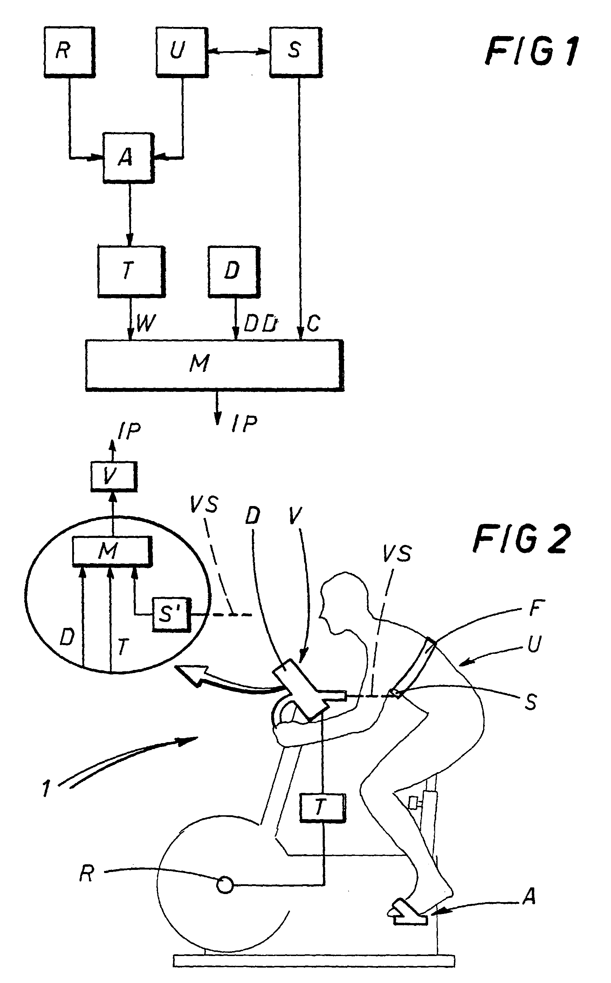

With reference to the accompanying drawings, a fitness machine with monitoring of the athlete's performance, denoted as a whole by 1 in figure 2, includes means A for the performance of an exercise by a user U, that is to say, means A for the application of force by the user or athlete U which have a resistance unit R designed to provide resistance to the force applied by the athlete.

In figure 2 the means A for the performance of an exercise are an exercise bike, in particular, the pedals with which the athlete U can apply a force countered by a resistance R produced by the fitness machine 1, for example, by means of an electromagnetic brake (of the known type, not illustrated).

The resistance unit R is connected to transducer means T, designed to supply as output a first signal W relative to the user's physical effort during exercise; in the embodiment shown in figure 2, the transducer means are schematically represented by a block T and, in practice, may consist of a device designed to transform the resistance of the electromagnetic brake into a value which is a function of the physical effort exerted by the athlete U.

Obviously, different types of transducers of physical effort are envisaged for different types of machines. For example, on a jogging machine, the transducer means will provide values correlated to the conveyor speed and angle.

Sensors S for the measurement of the heart rate are also envisaged, the sensors being applied to the user U during performance of the exercise and having means for the transmission of a second signal C relative to the user's heart rate.

In the embodiment illustrated, the sensors S consist of a frequency meter S supported by a band F attached to the user's chest U. This form of sensor is shown by way of example only, since other types of sensor may be used.

Data entry means D for variable user personal data DD are also envisaged. These data entry means may consist of a set of keys on a section of the machine 1, and may be linked to a monitor V, which can be used for the exchange of data between the machine 1 and the user U.

The data entered may be relative to the weight and age of the athlete U.

Finally, processing means M are connected to the input of the transducer means T, to the sensors S and the data entry means D.

The connections to the processing means M may be made using cables or radio waves, as shown in figure 2 for the sensors S, connected by a broken line VS to a receiver S' connected to the processing means M.

The processing means M may be of the type with a semiconductor and provide as output, by means of a preset algorithm, a value which is a function of the first signal W, the second signal C and the data DD, thus defining a variable IP relative to the physical performance of the user U.

The processing means M may use the first signal W and the second signal C, correlating them to a unit of time and the personal data DD of the athlete U.

For example, the first signal W, relative to the physical effort exerted by the athlete U, may be expressed in calories related to the time taken to perform the exercise and the athlete's weight.

In particular, the so-called METS unit of measurement may be used, corresponding to the ratio of calories consumed in one hour to the athlete's weight.

The METS unit of measurement can be used to ascertain oxygen consumption per unit of time, by multiplying the METS by 3.5.

Similarly, the heart rate measured by the sensors S can be better considered if related to the athlete's age.

The IP variable relative to the user's physical performance is a function of the physical effort exerted by the user during exercise, of the heart rate during exercise, and of the variable personal data DD of the user U.

In a possible embodiment, the machine 1 may envisage a variable IP equivalent to:

$$IP = 100 \times \{[(VO_2 / \%HR) - 0.1] / 0.9\}$$

where $VO_2$ corresponds to the product of 3.5 multiplied by the ratio of calories consumed during an hour of exercise to the weight of the user U (that is to say, the number of METS referred to above), whilst %HR corresponds to the ratio of the user's mean heart rate during exercise to the estimated maximum heart rate (equivalent to the difference between 220 and the user's age).

In this way, the athlete's heart rate is measured not as a data item in itself, but is related to the athlete's age.

The values 100, 0.1 and 0.9 in the above expression allow a number between 0 and 100 to be obtained, said number being easy to use and immediately understandable.

The following example renders the expression more comprehensible.

An exercise is performed for 20 minutes by a 20 year old, 70 Kg athlete, consuming 350 Kcalories.

The METS value is equivalent to the ratio of the calories consumed in one hour (350x3=1050) to the athlete's weight (70), that is to say, 15.

The corresponding volume of oxygen consumed during exercise ($VO_2$) is METS x 3.5 = 52.5 expressed in ml/min/Kg.

Moreover, during exercise, the sensors measure a mean heart rate equivalent to 150 beats per minute. Since the estimated maximum heart rate is 220 - 20 = 200 beats/minute, the %HR is equivalent to 150/200 = 75%.

The resulting IP value is:

$$100 \times \{[(52.5/75) - 0.1] / 0.9\} = 66.67$$

As already stated, the value is between 0 and 100 and allows an assessment of the athlete's level of fitness. The higher the value, the better the athlete's aerobic capacity. The value increases if the athlete improves his/her aerobic capacity, that is to say, consumes more oxygen at the same heart rate: in practice, with the same number of heart beats per minute he/she becomes capable of increased physical effort (in this case, being able to pedal against increased resistance produced by the machine), or if, following training, he/she loses weight, since the oxygen consumption capacity for each kilogram of weight will be greater.

A further advantage of the present invention is the fact that it allows a comparison between individuals of different ages, since the heart rate is not considered as an absolute factor, but rather the percentage heart rate which is a function of the age.

**Claims**

1.  A fitness machine with monitoring of the athlete's physical performance including:

    -   means (A) for the performance of an exercise by the user, constituted by means (A) for the application of force by the user (U) which have a resistance unit (R) designed to provide resistance to the force applied;
    -   transducer means (T), connected to the said resistance unit (R), designed to take a value of resistance produced by said resistance unit (R), and designed to provide as output a first signal (W) relative to the force applied by the user during exercise;
    -   sensor means (S) which measure the heart rate and can be applied to the user (U) during exercise, said sensor means having means for the transmission of a second signal (C) relative to the measurement of the heart rate;
    -   data entry means (D) for variable personal data (DD) of the user (U);
    -   processing means (M) connected to the input of the transducer means (T), to the sensors (S) which measure the heart rate and the data entry means (D), said processing means providing as output, by means of a preset algorithm, a value which is a function of the first signal (W), the second signal (C) and the data (DD), thus defining a variable (IP) relative to the physical performance of the user (U).

2.  The fitness machine as described in claim 1, **characterised in that** the said variable (IP), provided as output by the processing means (M), constitutes a value which is a function of the first and second signals (W, C) correlated to a unit of time.

3.  The fitness machine as described in claim 1, **characterised in that** the data (DD) received by the data entry means (D) relates at least to the weight and age of the user (U).

4.  The fitness machine as described in claim 1, **characterised in that** the variable (IP), relative to the user's physical performance, is a function of the physical effort exerted by the user during exercise, the heart rate during exercise, and the said variable personal data (DD) of the user (U).

5.  The fitness machine as described in claim 1, **characterised in that** the variable (IP), relative to the user's physical performance, is a function of the mean oxygen consumption by the user during exercise, the heart rate during training, and the said variable personal data (DD) of the user (U).

6.  The fitness machine as described in claim 1, **characterised in that** the variable (IP), relative to the user's physical performance, is defined by a value equivalent to the product of the number 100 multiplied by a first fraction with the denominator 0.9 and a numerator equivalent to the difference between a second fraction and the value 0.1, said second fraction having a numerator whose value corresponds to the mean oxygen consumption during exercise and a denominator which corresponds to the mean heart rate during exercise.

7.  The fitness machine as described in claim 1, **characterised in that** the variable (IP), relative to the user's physical performance, is defined by a value equivalent to the product of the number 100 multiplied by a first fraction with the denominator 0.9 and a numerator equivalent to the difference between a second fraction and the value 0.1, said second fraction having a numerator whose value corresponds to the mean oxygen consumption during exercise and a denominator which corresponds to the mean heart rate during exercise with relation to the age of the user.

8.  The fitness machine as described in claim 1, **characterised in that** the variable (IP), relative to the user's physical performance, is equivalent to:

    $$IP = 100 \times \{[(VO_2 \,/\, \%HR) - 0.1] \,/\, 0.9\}$$

    where $VO_2$ corresponds to the product of 3.5 multiplied by the ratio of the calories consumed during an hour of exercise to the weight of the user (U), and %HR corresponds to the ratio of the user's mean heart rate during exercise to the difference between 220 and the user's age.

**Patentansprüche**

1.  Fitnessgerät mit Überwachung des physischen Leistungsniveaus des Sportlers, enthaltend:

    -   Mittel (A) zum Durchführen eines Trainings durch den Benutzer, bestehend aus Mitteln (A) zum Anwenden einer Kraft durch den Benutzer (U), welche eine Widerstandeinheit (R) haben, die zum Erzeugen eines Widerstandes gegen die angewandte Kraft bestimmt ist;
    -   Umwandlungsmittel (T), die an die genannte Widerstandseinheit (R) angeschlossen und dazu bestimmt sind, einen durch die genannte Widerstandseinheit (R) erzeugten Widerstandswert aufzunehmen, sowie dazu bestimmt, im Ausgang ein erstes Signal (W) zu liefern, das sich auf die durch den Benutzer während des Trainings angewandte Kraft bezieht;

- Fühler (S), welche die Herzfrequenz messen und während des Trainings an den Benutzer (U) angeschlossen werden können, wobei die genannten Fühler Mittel zum Übertragen eines zweiten Signals (C) haben, welches sich auf die Messung der Herzfrequenz bezieht;
- Dateneingabemittel (D) für variable persönliche Daten (DD) des Benutzers (U);
- Verarbeitungsmittel (M), die im Eingang an die Umwandlungsmittel (T), an die Fühler (S), welche die Herzfrequenz messen, und an die Dateneingabemittel (D) angeschlossen sind, wobei die genannten Verarbeitungsmittel im Ausgang und mit Hilfe von einem festgelegten Algorithmus einen Wert liefern, welcher Funktion des ersten Signals (W), des zweiten Signals (C) und der Daten (DD) ist, wobei somit eine Variable (IP) beschrieben wird, die sich auf die physische Leistung des Benutzers (U) bezieht.

2. Fitnessgerät nach Patentanspruch 1, **dadurch gekennzeichnet, dass** die genannte, im Ausgang durch die Verarbeitungsmittel (M) gelieferte Variable (IP) einen Wert bildet, welcher Funktion des ersten und zweiten Signals (W, C) ist, bezogen auf eine Zeiteinheit.

3. Fitnessgerät nach Patentanspruch 1, **dadurch gekennzeichnet, dass** sich die von den Dateneingabemitteln (D) erhaltenen Daten (DD) wenigstens auf das Gewicht und das Alter des Benutzers (U) beziehen.

4. Fitnessgerät nach Patentanspruch 1, **dadurch gekennzeichnet, dass** die Variable (IP), die sich auf die physische Leistung des Benutzers bezieht, Funktion der von dem Benutzer während des Trainings angewandten physischen Kraft, der Herzfrequenz während des Trainings und der genannten variablen persönlichen Daten (DD) des Benutzers (U) ist.

5. Fitnessgerät nach Patentanspruch 1, **dadurch gekennzeichnet, dass** die Variable (IP), die sich auf die physische Leistung des Benutzers bezieht, Funktion des durchschnittlichen Sauerstoffverbrauch durch den Benutzer während des Trainings, der Herzfrequenz während des Trainings und der genannten variablen perönlichen Daten (DD) des Benutzers (U) ist.

6. Fitnessgerät nach Patentanspruch 1, **dadurch gekennzeichnet, dass** die Variable (IP), die sich auf die physische Leistung des Benutzers bezieht, beschrieben wird durch einen Wert äquivalent dem Produkt der Zahl 100, multipliziert mit einem ersten Bruch mit dem Nenner 0.9 und einem Zähler, welcher der Differenz zwischen einem zweiten Bruch und dem Wert 0.1 entspricht, wobei der genannte zweite Bruch einen Zähler hat, dessen Wert dem durchschnittlichen Sauerstoffverbrauch während des Trainings entspricht, und einen Nenner, welcher der mittleren Herzfrequenz während des Trainings entspricht.

7. Fitnessgerät nach Patentanspruch 1, **dadurch gekennzeichnet, dass** die Variable (IP), die sich auf die physische Leistung des Benutzers bezieht, beschrieben wird durch einen Wert äquivalent dem Produkt der Zahl 100, multipliziert mit einem ersten Bruch mit dem Nenner 0.9 und einem Zähler, welcher der Differenz zwischen einem zweiten Bruch und dem Wert 0.1 entspricht, wobei der genannte zweite Bruch einen Zähler hat, dessen Wert dem durchschnittlichen Sauerstoffverbrauch während des Trainings entspricht, und einen Nenner, welcher der mittleren Herzfrequenz während des Trainings im Verhältnis zu dem Alter des Benutzers entspricht.

8. Fitnessgerät nach Patentanspruch 1, **dadurch gekennzeichnet, dass** die Variable (IP), die sich auf die physische Leistung des Benutzers bezieht, äquivalent ist mit:

$$IP = 100 \times \{[(V02/\%HR) - 0.1]/0.9\}$$

- wo V02 dem Produkt von 3.5 entspricht, multipliziert mit dem Verhältnis zwischen den während einer Trainingsstunde verbrauchten Kalorien und dem Gewicht des Benutzers (U), und %HR entspricht dem Verhältnis zwischen der mittleren Herzfrequenz des Benutzers während des Trainings und der Differenz zwischen 220 und dem Alter des Benutzers.

**Revendications**

1. Un appareil d'exercice avec évaluation de la performance physique de l'athlète comprenant :

- des moyens (A) pour l'exécution d'un exercice par l'utilisateur, constitués par des moyens (A) pour l'application d'une force par l'utilisateur (U) qui sont pourvus d'une unité de résistance (R) destinée à fournir une résistance à la force appliquée ;
- des moyens transducteurs (T), reliés à ladite unité de résistance (R), destinés à prendre une valeur de résistance produite par cette même unité de résistance (R), et destinés à fournir en sortie un premier signal (W) relatif à la force appliquée par l'utilisateur durant l'exercice ;

- des moyens capteurs (S) qui mesurent la fréquence cardiaque et peuvent être appliqués sur l'utilisateur (U) durant l'exercice, lesdits moyens capteurs étant pourvus de moyens pour la transmission d'un deuxième signal (C) relatif à la mesure de la fréquence cardiaque ;
- des moyens de saisie de données (D) pour les données personnelles variables (DD) de l'utilisateur (U) ;
- des moyens de traitement (M) reliés en entrée aux moyens transducteurs (T), aux capteurs (S) qui mesurent la fréquence cardiaque et aux moyens de saisie de données (D), lesdits moyens de traitement fournissant en sortie, par le biais d'un algorithme préétabli, une valeur qui est fonction du premier signal (W), du deuxième signal (C) et des données (DD), définissant ainsi une variable (IP) relative à la performance physique de l'utilisateur (U).

2. L'appareil d'exercice selon la revendication 1, **caractérisé en ce que** ladite variable (IP), fournie en sortie par les moyens de traitement (M), constitue une valeur qui est fonction des premier et deuxième signaux (W, C) corrélés à une unité de temps.

3. L'appareil d'exercice selon la revendication 1, **caractérisé en ce que** lesdites données (DD) reçues par les moyens de saisie de données (D) se réfèrent au moins au poids et à l'âge de l'utilisateur (U).

4. L'appareil d'exercice selon la revendication 1, **caractérisé en ce que** ladite variable (IP), relative à la performance physique de l'utilisateur, est fonction de l'effort physique exercé par l'utilisateur pendant l'exercice, de la fréquence cardiaque pendant ce même exercice et des données personnelles variables (DD) susmentionnées de l'utilisateur (U).

5. L'appareil d'exercice selon la revendication 1, **caractérisé en ce que** ladite variable (IP), relative à la performance physique de l'utilisateur, est fonction de la consommation d'oxygène moyenne de la part de l'utilisateur pendant l'exercice, de la fréquence cardiaque pendant l'entraînement et des données personnelles variables (DD) susmentionnées de l'utilisateur (U).

6. L'appareil d'exercice selon la revendication 1, **caractérisé en ce que** ladite variable (IP), relative à la performance physique de l'utilisateur, est définie par une valeur qui équivaut au produit du nombre 100 multiplié par une première fraction ayant le dénominateur 0,9 et un numérateur équivalant à la différence entre une deuxième fraction et la valeur 0,1, ladite deuxième fraction ayant un numérateur dont la valeur correspond à la consommation d'oxygène moyenne pendant l'exercice et un dénominateur qui correspond à la fréquence cardiaque moyenne pendant ce même exercice.

7. L'appareil d'exercice selon la revendication 1, **caractérisé en ce que** ladite variable (IP), relative à la performance physique de l'utilisateur, est définie par une valeur qui équivaut au produit du nombre 100 multiplié par une première fraction ayant le dénominateur 0,9 et un numérateur équivalant à la différence entre une deuxième fraction et la valeur 0,1, ladite deuxième fraction ayant un numérateur dont la valeur correspond à la consommation d'oxygène moyenne pendant l'exercice et un dénominateur qui correspond à la fréquence cardiaque moyenne pendant ce même exercice rapportée à l'âge de l'utilisateur.

8. L'appareil d'exercice selon la revendication 1, **caractérisé en ce que** ladite variable (IP), relative à la performance physique de l'utilisateur, est équivalente à :

$$IP = 100 \times \{[(VO_2 / \%HR) - 0,1] / 0,9\}$$

où $VO_2$ correspond au produit de 3,5 multiplié par le rapport entre les calories consommées en une heure d'exercice et le poids de l'utilisateur (U), et où %HR correspond au rapport entre la fréquence cardiaque moyenne de l'utilisateur pendant l'exercice et la différence entre 220 et l'âge de l'utilisateur.

*FIG 1*

*FIG 2*